# FASCICULE DE BREVET EUROPEEN

(11) **EP 0 973 565 B1**
(45) Date de publication et mention de la délivrance du brevet: **10.11.2004**
(21) Numéro de dépôt: 97952096.2
(22) Date de dépôt: 18.12.1997
(51) Int. Cl.: A61M 5/00, A61M 5/142

(54) **DISPOSITIF MEDICAL D'INJECTION DE LIQUIDE**
MEDIZINISCHE VORRICHTUNG ZUM INJIZIEREN VON FLÜSSIGKEITEN
MEDICAL DEVICE FOR INJECTING LIQUID

(30) Priorité: 18.12.1996 FR 9615551
(43) Date de publication de la demande: 26.01.2000
(73) Titulaire: SMC-Swiss Medical Care S.A., 1004 Lausanne (CH)
(72) Inventeur: NEFTEL, Frédéric, CH-1003 Lausanne (CH)
(74) Mandataire: Dronne, Guy
(86) Numéro de dépôt international: PCT/FR1997/002341
(87) Numéro de publication internationale: WO 1998/026818

(56) Documents cités:
- EP-A- 0 648 513
- EP-A- 0 650 739
- US-A- 5 533 978
- US-A- 5 569 181
- US-A- 5 569 208

## Description

La présente invention se rapporte à un dispositif médical d'injection de liquide.

Un tel dispositif peut par exemple trouver une application pour une pompe utilisée pour l'injection de liquide de contraste pour l'imagerie médicale.

Un problème majeur résultant de l'injection de liquide à des patients est le risque de contamination du dispositif d'injection par le patient. En effet, lorsque l'on administre un liquide à un patient; il existe un risque de reflux du liquide injecté alors que ce dernier est déjà entré en contact avec le patient. De même, en l'absence d'injection, il existe un risque de contamination par migration des agents contaminants, tels que germes du patient, vers le dispositif d'injection. Une telle situation rend obligatoire, lorsque l'on traite un nouveau patient, le plus souvent, le changement de toutes les parties du dispositif qui ont été utilisées.

Il existe plusieurs dispositifs de l'art antérieur qui permettent de diminuer le risque de reflux : EP-A-648513 de la société MEDEX S.A. décrit, par exemple une unité d'injection qui comprend une valve anti-retour. Le document US-A-5.569.208 concerne un système de gestion de délivrance de liquide qui utilise deux valves unidirectionnelles 39 et 34 disposées en série en tant que double système visant à protéger la poche de liquide 12. EP-A-279028, des sociétés KABI PHARMACIA GmbH et PFRIMMER-VIGGO GmbH, revendique un dispositif d'injection de liquide comprenant une valve anti-retour caractérisée par le fait que la pression d'ouverture peut être réglée entre 2 positions.

Bien que les dispositifs de l'art antérieur diminuent le risque de reflux, il subsiste toujours un risque de fuite de la valve anti-retour. Ce risque est particulièrement élevé lorsqu'il y absence d'injection.

La présente invention vise donc à éliminer totalement le risque de reflux. De plus, la présente invention vise également à permettre de détecter toute fuite des moyens de sécurité mis en place pour éviter ce reflux, si ce dernier devait quand même avoir lieu.

A cet effet, on utilise un dispositif médical d'injection de liquide comprenant une tubulure sur laquelle se situe au moins un premier système d'occlusion du liquide tel qu'une valve anti-retour. De plus, un système de régulation se situe en amont du premier système d'occlusion, les 2 systèmes définissant un segment intermédiaire dans lequel la pression, en absence d'injection, est supérieure à la pression régnant en aval du premier système d'occlusion. De cette façon, toute fuite de liquide au niveau du premier système d'occlusion est orientée vers l'aval et toute fuite depuis le segment intermédiaire peut être détectée par mesure de la chute de pression associée.

Les termes aval et amont signifient respectivement en direction du patient et en direction opposée.

Le système de régulation peut par exemple être une deuxième valve anti-retour ou un système d'écrasement de la tubulure tel que les galets de la cassette péristaltique correspondant au brevet FR 8903234 de la société MALBEC S.A.

Il faut remarquer que le système de régulation ne peut être considéré comme une simple sécurité supplémentaire, indépendante du premier système d'occlusion.

Bien au contraire, les 2 systèmes constituent un ensemble interactif car la présence du système de régulation permet d'instaurer, éventuellement de maintenir, une pression positive entre les 2 systèmes qui empêche toute fuite éventuelle de liquide de l'aval du premier système d'occlusion en direction du segment intermédiaire.

Il faut en outre relever que la pression entre les 2 systèmes est constante en l'absence de fuite car l'espace entre les 2 systèmes est rempli par du liquide qui, à l'instar de tout autre liquide, est incompressible.

Afin d'augmenter la sécurité du dispositif, il est également souhaitable d'avoir des systèmes de nature différente. De la sorte, si un système fait défaut, le deuxième pourra tout de même agir d'une façon similaire à ce qui est décrit dans l'art antérieur.

De plus, avec des systèmes de nature différente, et plus précisément avec des systèmes caractérisés par le fait qu'ils ont chacun une pression d'ouverture différente, il est possible d'orienter le sens du liquide lorsqu'il y a fuite ou pression élevée dans le segment intermédiaire.

En particulier, si la pression d'ouverture du premier système d'occlusion est inférieure à la pression d'ouverture du système de régulation, tout mouvement de liquide lors de l'interruption de l'injection se fera vers l'aval, le système de régulation se fermant avant le premier sytème d'occlusion, évitant par la même toute contamination des parties du dispositif situées en amont du premier système d'occlusion.

De même, en cas de pression élevée dans le segment intermédiaire, le premier système d'occlusion sera le premier susceptible de s'ouvrir en cas de fuite, orientant ainsi le liquide en aval du premier système d'occlusion et empêchant tout reflux vers le segment intermédiaire.

Il est également possible d'envisager que les 2 pressions d'ouverture des systèmes de régulation soient identiques, mais cela ne peut se concevoir que si la pression d'ouverture est supérieure à la pression régnant en aval du premier système d'occlusion lorsqu'il y a absence d'injection.

En outre, il est souhaitable d'avoir une pression d'ouverture du premier système d'occlusion qui soit supérieure à la pression maximale pouvant s'instaurer en aval de celui-ci du fait du patient en l'absence d'injection. Par ailleurs, en absence d'injection, il est nécessaire que la pression d'ouverture du premier système d'occlusion soit supérieure à la pression du segment intermédiaire.

Dans la plupart des cas, en absence d'injection, la pression régnant en aval du premier système d'occlusion correspond à la pression veineuse du patient.

Dans un autre mode préférentiel, il est souhaitable de munir la tubulure d'un système de déconnexion situé entre les 2 systèmes de régulation. Le système de déconnexion définissant en aval une tubulure à usage unique et en amont une tubulure à usage multiple.
Ainsi, dans le cas où la tubulure est contaminée uniquement en aval du système de déconnexion, il est possible de changer seulement cette partie du dispositif et de réutiliser le reste (système de pompage, cassette péristaltique, réservoir, etc...) pour d'autres patients.

De préférence, le système de déconnexion est situé le plus près possible du deuxième dispositif de régulation ou, ce qui revient au même, le plus loin possible de la valve anti-retour. Ainsi, dans le cas fort improbable où une contamination se propage vers l'amont, le risque de contaminer la zone située en amont du système de déconnexion est minimisé car le trajet à effectuer jusqu'au système de déconnexion est maximisé.

Dans un autre mode préférentiel, le système de déconnexion est muni de moyens d'occlusion de la tubulure qui s'activent préalablement à la déconnexion, ce qui évite tout risque d'éjection du liquide de la tubulure qui se trouve sous pression. Un tel système de fermeture automatique est par exemple décrit dans les brevets US-A-5549566 et US-A-5533996 des sociétés ABBOTT LAB et BAXTER INT INC respectivement.
Les moyens d'occlusion précités peuvent également être conçus de façon à s'ouvrir lors de la connexion.

De tels systèmes de déconnexion peuvent par exemple être de type "luer-lock" couplés à un système de robinet rotatif sur chacun des deux segments connectés. En outre, le système de connexion entre les deux pièces peut être de type clef-serrure, n'autorisant ainsi que la connexion de deux éléments correctement codés l'un par rapport à l'autre. Un tel système rotatif permet d'ouvrir et fermer les deux robinets placés de part et d'autre de la connexion lors de la rotation de la clef dans la serrure, tout en empêchant la déconnexion des deux pièces lorsque la clef est engagée dans la position correspondant à la position ouverte des systèmes d'occlusion.

Le système d'occlusion situé dans le segment aval peut également être une valve anti-retour et le système dans le segment amont un clapet ouvert lorsque les deux pièces sont engagées ou, alternativement, les deux systèmes d'occlusion peuvent être des clapets ouverts uniquement lorsque les deux pièces sont engagées.

Dans un autre mode préférentiel, un capteur de pression se situe sur le segment intermédiaire. De la sorte, il est possible de détecter toute variation de pression dans cette zone.
Dans le cas où le capteur de pression rentre en contact avec la tubulure, il est avantageux de le placer entre le système de déconnexion et le système de régulation; cette configuration ayant le mérite de pouvoir réutiliser la même configuration du capteur de pression pour plusieurs patients.

En l'absence d'injection, toute chute de pression entre les 2 systèmes implique la présence d'une fuite qui, très vraisemblablement, peut se produire au niveau du premier système d'occlusion ou au niveau du système de régulation.

Si la fuite a lieu au niveau du premier système d'occlusion, la contamination peut se propager dans la tubulure à usage multiple.

Si la fuite a lieu au niveau du système de régulation, il n'y a pas obligatoirement propagation de la contamination dans la tubulure à usage multiple mais, par contre, un tel risque augmente car toute fuite au niveau du premier système d'occlusion mènerait à une baisse de pression du segment intermédiaire supprimant ainsi tout l'effet des moyens mis en place dans le cadre de la présente invention.

Afin de palier au deux cas de figures précédemment décrits, il est avantageux d'associer une alarme au capteur de pression qui est placé sur le segment intermédiaire. De plus, de manière encore plus avantageuse, l'injection de liquide peut s'enclencher de façon à rétablir la pression initiale lorsque l'alarme est activée.

Il faut également noter que le maintien d'une pression élevée dans le segment intermédiaire, proche de la pression d'ouverture du premier système d'occlusion, peut être de nature à favoriser la fuite au niveau de ce premier système d'occlusion.

A cet effet, on prévoit des moyens pour faire diminuer la pression dans le segment intermédiaire. Ces moyens peuvent être constitués d'une chambre intermédiaire de volume réglable. Le réglage du volume de la chambre intermédiaire peut par exemple être effectué par un piston. Ainsi la pression régnant dans le segment intermédiaire peut être choisie pour être supérieure à la pression en aval du premier système d'occlusion et suffisamment éloignée de la pression d'ouverture de ce premier système d'occlusion afin d'utiliser pleinement les caractéristiques d'étanchéité de ce premier système d'occlusion. Un autre moyen permettant de faire baisser cette pression en dessous du seuil d'ouverture du premier système d'occlusion consiste à choisir un système de régulation dont l'ouverture peut être réglée de façon à abaisser la pression du segment intermédiaire jusqu'à une valeur prédéterminée.

Bien que le réglage de la pression dans le segment intermédiaire minimise considérablement le risque de contamination, il est toujours possible, lorsque l'alarme se déclenche, de procéder au changement des parties susceptibles d'avoir été contaminées telles que par exemple la cassette péristaltique. Il est dès lors avantageux de prévoir à cet effet un deuxième système de déconnexion en amont du système de régulation.

Optionnellement, un deuxième capteur de pression peut être placé sur le segment de tubulure qui se trouve en aval du premier système d'occlusion, ce qui permet par exemple de détecter toute surpression par rapport à la pression du segment intermédiaire.

On peut également munir le dispositif d'un système de mesure de la différence de pression mesurée par les 2 capteurs, ce qui a pour avantage de déterminer plus directement le risque de reflux. Une alarme peut se déclencher lorsqu'il y a tendance à l'égalisation des pressions situées en aval et en amont de la valve anti-retour.

Le dispositif médical selon l'invention peut également être conçu de façon à désactiver l'alarme lorsque la tubulure à usage unique est déconnectée.

Le dispositif médical selon l'invention peut avantageusement inclure un pousse-seringue. Un capteur de pression peut être placé sur l'extrémité distale du piston du pousse seringue.

Dans l'hypothèse de l'utilisation d'un pousse-seringue, il est possible de considérer la seringue elle-même comme partie intégrante du segment intermédiaire, la seringue pouvant former elle-même le système de régulation. En outre, dans ce cas de figure, la seringue peut également faire office système de régulation de chambre intermédiaire permettant de régler la pression du segment intermédiaire.

Le dispositif médical selon l'invention peut être utilisé pour n'importe quel type de liquide, il peut notamment être utilisé pour l'injection de liquides de contraste.

L'invention est décrite ci-après à l'aide d'un exemple et de références aux dessins joints, dans lesquels :
- la figure 1 représente schématiquement un mode préférentiel de l'invention ;
- les figures 2A à 2I représentent les différentes étapes de fonctionnement d'un système de déconnexion préférentiel dans le cas de sa première utilisation ;

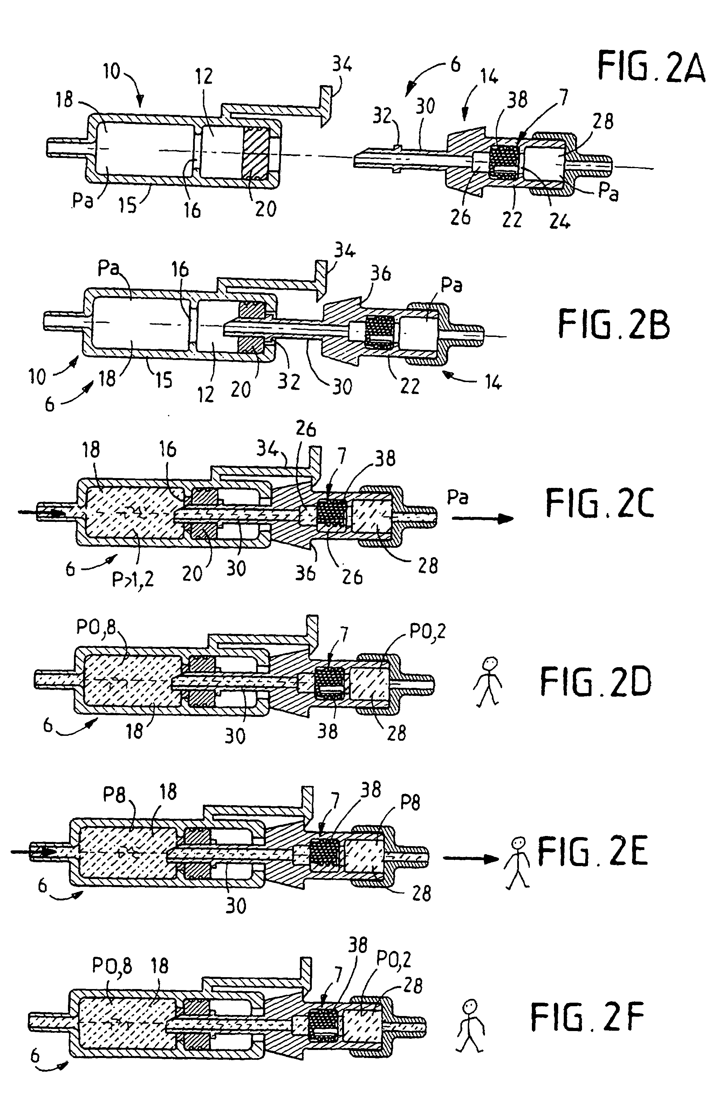

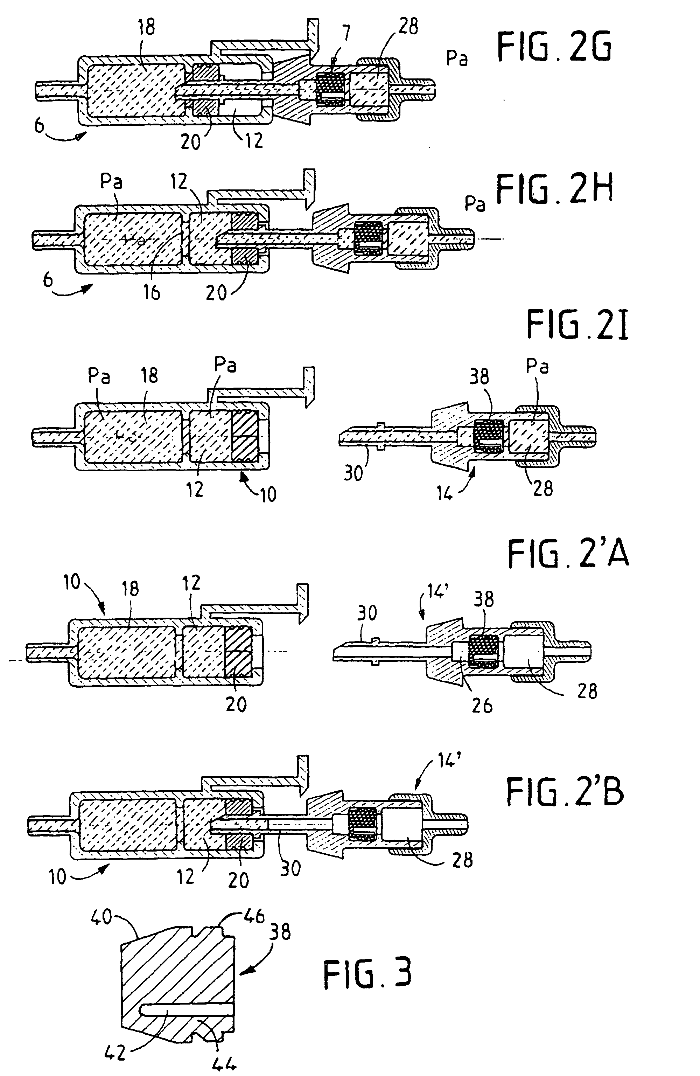

- les figures 2'A et 2'B correspondent aux étapes des figures 2A et 2B dans le cas des utilisations ultérieures du système de déconnexion ;
- la figure 3 illustre la section en coupe longitudinale d'un mode préférentiel de réalisation d'une valve anti-retour, et

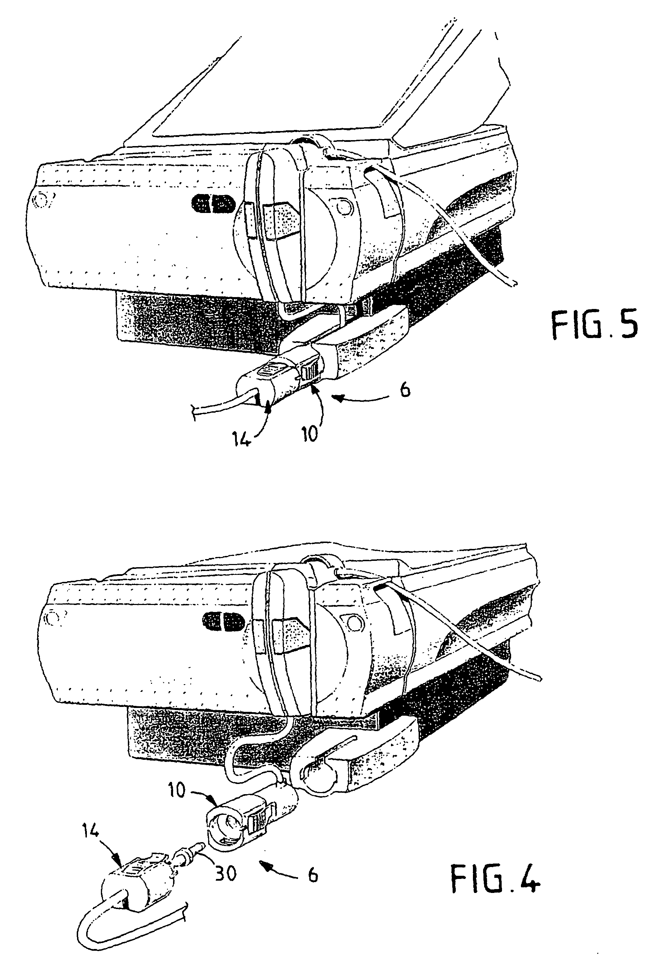

- les figures 4 et 5 illustrent, en perspective, le système de déconnexion des figures 2A à 2I, 2'A et 2'B, respectivement avant et après son branchement sur un dispositif d'injection de liquide, en aval d'une cassette péristaltique.

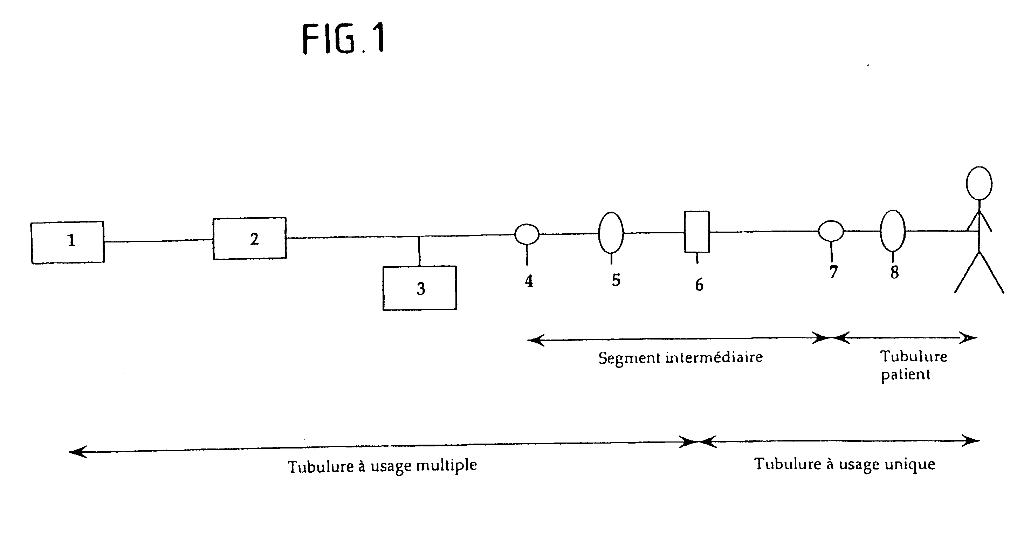

Le dispositif d'injection de liquide (figure 1) est constitué essentiellement d'une tubulure, d'un réservoir 1, d'un système de pompage 2, d'une chambre intermédiaire 3 de volume variable, d'une cassette péristaltique 4 et d'une valve anti-retour 7. Un système de déconnexion 6 est situé en amont de la valve anti-retour 7, entre cette dernière et la cassette péristaltique. Des capteurs de pression 5 et 8 sont situés en aval et en amont de la valve anti-retour.

En absence d'injection, c'est à dire lorsque la pompe est désactivée, il règne une pression constante entre la valve anti-retour et le deuxième système de régulation. La pression constante est inférieure à la pression d'ouverture de la valve anti-retour.

Si la valve anti-retour est défectueuse, le reflux du liquide en direction de la pompe est empêché par le flux du liquide lié à la pression régnant dans le segment intermédiaire, cette pression du segment intermédiaire étant due à la présence de la cassette péristaltique qui, en l'absence d'injection, occlut très efficacement la tubulure. La pression d'ouverture du système d'occlusion constitué par les galets de la cassette péristaltique est supérieure à 8 bar, alors que la pression d'ouverture du premier système d'occlusion est égale à 0,5 bar.

La présente invention ne se limite bien entendu pas seulement à l'exemple précédemment décrit, tous les cas de figure de la description sont également inclus.

Ainsi, de préférence, le premier système d'occlusion ou le système de régulation, ou les deux, sont de type clamp automatique. Il est également possible d'utiliser un quatrième dispositif d'occlusion, de préférence placé en aval du système de déconnexion, qui est activé en cas de déclenchement de l'alarme.

Le dispositif selon la présente invention peut comprendre en outre un capteur de pression mesurant la pression de liquide dans un tronçon de la tubulure situé en amont du segment intermédiaire.

Si l'on se reporte à la figure 2A, un système de déconnexion 6 préférentiel comprend une pièce amont 10 avec une chambre intermédiaire 12 de volume réglable et une pièce aval 14 munie de la valve anti-retour 7 et pouvant coopérer de manière réversible avec la pièce amont 10 pour former une liaison étanche.

La pièce amont 10 est formée d'un corps 15 tubulaire creux ouvert à ses deux extrémités et délimitant un volume interne divisé en deux par une paroi radiale intérieure ouverte 16 placée entre une chambre d'injection 18 et la chambre intermédiaire 12 de volume réglable, constituant une chambre de décompression comme il sera expliqué ci-après.

La chambre intermédiaire 12 contient un septum élastique préfendu 20 dont le diamètre est sensiblement égal au diamètre intérieur de la chambre intermédiaire 12 afin de former un piston mobile en translation.

La pièce aval 14 comprend un corps de révolution 22 creux prolongé à ses deux extrémités ouvertes par une tige creuse. Le corps 22 enveloppe un volume intérieur divisé en deux par une paroi radiale ouverte 24 placée entre une première chambre 26, dans laquelle est formée la valve anti-retour 7, et une deuxième chambre 28 tournée en direction du patient.

La tige creuse 30 adjacente à la première chambre 26 présente une extrémité libre, dont la forme comprend une portion tronconique, et est destinée à se placer à l'intérieur de la chambre intermédiaire 12 en traversant le septum 20 et l'ouverture de la paroi 16. La tige creuse 30 comporte une collerette annulaire externe 32 venant en butée contre le septum 20 afin d'entraîner celui-ci en translation dans la chambre intermédiaire 12 (figure 2B), le septum 20 venant en butée contre la paroi 16 lorsque la tige 30 est enfoncée au maximum dans la pièce 10, le corps tubulaire 15 venant alors en butée contre le corps 22 (figure 2C).

Il est prévu en outre des moyens de verrouillage entre les pièces 10 et 14, par exemple sous la forme d'une patte élastique de verrouillage située à l'extérieur du corps 15 et dont l'extrémité libre vient s'encliqueter sur un épaulement ou une gorge de la surface extérieure du corps 22. Sur la figure 2B sont représentés une telle patte 34 associée à un épaulement tourné en direction opposée à la tige creuse 30 lorsque les corps 15 et 22 sont en butée.

La valve anti-retour 7 représentée est formée d'un bouchon 38 pouvant s'écraser sur lui-même radialement en permettant son contournement par le liquide lorsque la pression de liquide en amont de ce bouchon 38 est supérieure ou égale à la pression d'ouverture de la valve anti-retour 7.

Ce type de bouchon est préférentiellement du type de celui qui est décrit dans le brevet américain 4 929 230 c'est-à-dire un bouchon fabriqué dans un matériau élastique et dont la section longitudinale est visible sur les figures 2A à 2I et 2'A, 2'B et de façon encore plus précise sur la figure 3. Ce bouchon 38 possède une première extrémité orientée vers la deuxième chambre 28 avec des moyens d'étanchéité 46 coopérant avec la surface interne de la deuxième chambre 28, une deuxième extrémité orientée vers la tige creuse 30, au moins une portion de surface tronconique 40 entre les moyens d'étanchéité et la deuxième extrémité, et une cavité 42 s'étendant à l'intérieur dudit bouchon à partir de ladite première extrémité et sur une profondeur supérieure à la distance séparant les moyens d'étanchéité du bord libre de la première extrémité. Ce bouchon 38 possède en outre une paroi rétractable 44 entre au moins une partie de ladite surface tronconique 40 et ladite cavité 42, ladite paroi rétractable 44 s'étendant au moins depuis le fond de ladite cavité 42 jusqu'à ladite première extrémité et se rétractant dans ladite cavité 42 quand la résultante radiale de la pression exercée sur ladite surface tronconique 40 par ledit liquide est supérieure à la somme de la résistance à la compression radiale de la paroi rétractable 44 et de la pression interne de la cavité 42.

Ce type de bouchon 38 peut également être placé dans la tubulure en tant que deuxième système d'occlusion 4.

Selon un mode préférentiel de réalisation, la pression d'ouverture de la valve anti-retour 7 est supérieure à sa pression de fermeture. On pourra, par exemple, conformer le bouchon 38 pour qu'il s'écrase, en réalisant ainsi l'ouverture de la valve 7, à partir d'une pression de 1, 2 bar de liquide dans la tige creuse 30, la paroi rétractable 44 reprenant sa forme initiale, en réalisant ainsi la fermeture de la valve 7, pour une pression de liquide dans la tige creuse égale ou inférieure à 0,8 bar.

Avant toute injection (figures 2A et 2B), les pièces 10, 14 du système de connexion 6 sont vides de liquides et c'est la pression atmosphérique (Pa) qui règne.

D'une manière générale, dans toute la suite de la description, lorsque cette pression atmosphérique Pa n'est pas mentionnée, il faut considérer qu'elle s'ajoute à la pression de liquide indiquée.

Après connexion entre les pièces 10 et 14, on amorce le dispositif d'injection (figure 2C) en envoyant du liquide à une pression supérieure à 1,2 bar (P > 1, 2) dans la chambre d'injection 18. Ce liquide passe dans la tige creuse 30, le septum 20 fermant de façon étanche la chambre intermédiaire 12 qui présente un volume sensiblement nul en communication avec la chambre d'injection 18. Du fait de cette pression de 1, 2 bar supérieure à la pression d'ouverture de la valve 7, cette dernière est ouverte et le liquide peut traverser la première chambre 26 et sortir de la deuxième chambre 28 en communication avec l'extérieur (Pa).

L'étape suivante (figure 2D) consiste à arrêter l'envoi de liquide sous pression, la chambre d'injection 18 étant alors remplie d'un liquide sous une pression d'environ 0,8 bar (P0,8) entraînant la fermeture de la valve 7 par retour de la paroi rétractable 44 dans sa position initiale.

La valve 7 s'étant refermée, on branche ensuite le dispositif d'injection sur le patient de sorte que la pression veineuse du patient proche de 0,2 bar (P0,2) correspond sensiblement à la pression de liquide dans la deuxième chambre 28.

Pendant l'injection (figure 2E), la pression de liquide au niveau du système de déconnexion 6 est supérieure à 1,2 bar (P8) ce qui maintient la valve 7 ouverte.

Lorsque l'injection est terminée (figure 2F) la situation du système de déconnexion 6 est identique à celle (figure 2D) précédant l'étape d'injection (figure 2E).

Lorsqu'on débranche (figure 2G) le patient du dispositif d'injection, la valve 7 reste fermée, la pression de liquide dans la deuxième chambre 28 étant sensiblement nulle (hormis la pression atmosphérique) et la pression de liquide dans la chambre d'injection 18 étant sensiblement de l'ordre de 0,8 bar.

Ainsi, lorsqu'on écarte entre elles les pièces 10, 14 du système de déconnexion 6 (figure 2H), au fur et à mesure que la tige creuse 30 se retire et que la collerette 32 s'écarte de la paroi 16, le septum 20 s'écarte également de la paroi 16 car la pression de liquide dans la chambre d'injection 18 est supérieure à celle régnant dans la deuxième chambre 28.

Le volume des chambres 12 et 18 est prévu pour permettre au septum 20 de s'écarter suffisamment de la paroi 16 pour que la pression dans les chambres 12 et 18 tombe au niveau de la pression atmosphérique de sorte que l'on peut séparer les deux pièces 10 et 14 (figure 21) sans risque d'écoulement du liquide hors de la deuxième chambre 28 ou hors de la tige creuse 30. On comprend que le volume interne de ladite pièce amont 10 est conformé pour permettre d'obtenir une pression de liquide dans le système de déconnexion 6 qui soit inférieure ou égale à la pression atmosphérique avant la séparation complète entre lesdites pièces amont et aval 10, 14.

Grâce à ce système de déconnexion, le bouchon 38 évite le reflux en amont du liquide ayant rempli la deuxième chambre 28 et communicant avec la tubulure reliée au patient. De plus, tout écoulement de liquide est évité lors de la déconnexion entre les pièces 10 et 14.

Lors de l'utilisation (non revendiquée) du dispositif d'injection sur un nouveau patient, on utilise une nouvelle pièce aval 14' qui vient se connecter sur la pièce amont 10 déjà utilisée pour un patient précédent (figures 2'A et 2'B). Les étapes ultérieures pour la réalisation de l'injection sont identiques aux étapes illustrées sur les figures 2C à 2I déjà décrites.

## Revendications

1. Dispositif médical d'injection de liquide sous pression comprenant une source de liquide (1), des moyens de pompage (2), une tubulure sur laquelle se situent au moins un premier système d'occlusion du liquide présentant une première pression d'ouverture (P1) et; comprenant une valve (7) ou un clamp, et un système de régulation (4) présentant une deuxième pression d'ouverture (P2) et; placé en amont du premier système d'occlusion, et des moyens d'injection pouvant être reliés à un patient, **caractérisé par le fait que** le système de régulation (4) et le premier système d'occlusion du liquide (7) définissent un segment intermédiaire dans lequel la pression, en absence d'injection, est maintenue toujours supérieure à la pression régnant en aval du premier système d'occlusion (7), ladite première pression d'ouverture (P1) étant inférieure à ladite deuxième pression d'ouverture (P2) et la pression du segment intermédiaire étant inférieure à ladite première pression d'ouverture (P1) en l'absence d'injection, de façon à empêcher un reflux de liquide vers le segment intermédiaire au niveau du premier système d'occlusion (7).

2. Dispositif médical d'injection de liquide selon la revendication 1, **caractérisé par le fait que** toute fuite de liquide depuis le segment intermédiaire est détectée par mesure de la chute de pression associée.

3. Dispositif médical selon l'une quelconque des revendications 1 et 2, **caractérisé par le fait que** ladite valve ou ledit clamp du premier système d'occlusion du liquide est une valve anti-retour (7).

4. Dispositif médical selon la revendication 3, **caractérisé en ce que** la valve anti-retour (7) comprend un bouchon (38) pouvant s'écraser sur lui-même radialement en permettant son contournement par le liquide lorsque la pression de liquide en amont du bouchon (38) est supérieure ou égale à la pression d'ouverture de la valve anti-retour (7).

5. Dispositif médical selon l'une des revendications 3 ou 4, **caractérisé en ce que** la valve anti-retour (7) présente une pression d'ouverture supérieure à sa pression de fermeture.

6. Dispositif médical selon l'une quelconque des revendications 1 à 5, **caractérisé par le fait que** le système de régulation (4) comporte un deuxième système d'occlusion.

7. Dispositif médical selon la revendication 6, **caractérisé par le fait que** les premier et deuxième systèmes d'occlusion (4, 7) ont des pressions d'ouverture différentes.

8. Dispositif médical selon la revendication 7, **caractérisé par le fait que** la pression d'ouverture du deuxième système d'occlusion (4) est supérieure à la pression d'ouverture du premier système d'occlusion (7) afin d'assurer en permanence un flux de liquide vers l'aval.

9. Dispositif médical selon l'une quelconque des revendications 6 à 8, **caractérisé par le fait que** le deuxième système d'occlusion (4) est un système d'écrasement de la tubulure.

10. Dispositif médical selon la revendication 9, **caractérisé par le fait que** le système d'écrasement de la tubulure est constitué par les galets d'une cassette péristaltique.

11. Dispositif médical selon l'une quelconque des revendications 6 à 8, **caractérisé par le fait que** le deuxième système d'occlusion (4) comprend une valve anti-retour.

12. Dispositif médical selon la revendication 11, **caractérisé en ce que** la valve anti-retour (7) comprend un bouchon (38) pouvant s'écraser sur lui-même radialement en permettant son contournement par le liquide lorsque la pression de liquide en amont du bouchon (38) est supérieure ou égale à une pression d'ouverture prédéterminée.

13. Dispositif médical selon l'une quelconque des revendications précédentes, **caractérisé par le fait que** la pression d'ouverture du premier système d'occlusion (7) est supérieure à la pression régnant dans le segment intermédiaire en absence d'injection.

14. Dispositif médical selon l'une quelconque des revendications précédentes, **caractérisé par le fait qu'**il comprend en outre un système de déconnexion (6) se situant sur le segment intermédiaire, le système de déconnexion (6) définissant en aval une tubulure à usage unique et en amont une tubulure à usage multiple.

15. Dispositif médical selon la revendication 14, **caractérisé par le fait que** le système de déconnexion (6) est muni de moyens d'occlusion (7, 20) de la tubulure qui s'activent préalablement à la déconnexion.

16. Dispositif médical selon la revendication 15, **caractérisé par le fait que** le système de déconnexion comporte deux pièces codées l'une par rapport à l'autre qui ne permettent l'activation ou la désactivation des moyens d'occlusion que si le codage d'une pièce par rapport à l'autre est respecté.

17. Dispositif médical selon la revendication 16, **caractérisé par le fait que** le codage est de type clef-serrure.

18. Dispositif médical selon la revendication 17, **caractérisé par le fait que** l'activation, la désactivation des moyens d'occlusion est assurée par une rotation de telle sorte que la déconnexion ne puisse être faite lorsque la clef est tournée dans la serrure de telle façon que les moyens d'occlusion sont désactivés et que le retour de la clef dans la position de libération entraîne la fermeture des moyens d'occlusion.

19. Dispositif médical selon la revendication 15, **caractérisé par le fait que** le système de déconnexion (6) comporte deux pièces (10, 14) équipées chacune de moyens d'occlusion (20, 7), les moyens d'occlusion de l'une des deux pièces au moins étant de type clapet (7) et s'ouvrant automatiquement lorsque les deux pièces (10, 14) sont engagées de façon étanche et que la pression de liquide en amont est supérieure à la pression d'ouverture dudit clapet (7).

20. Dispositif médical selon l'une quelconque des revendications précédentes, **caractérisé par le fait qu'**un premier capteur de pression (5) permet de mesurer la pression de liquide et/ou la variation de pression de liquide au niveau du segment intermédiaire.

21. Dispositif médical selon la revendication 20 et l'une des revendications 14 ou 15, **caractérisé par le fait que** le capteur de pression (5) est situé en amont du système de déconnexion (6) de façon à être en contact avec une partie de la tubulure à usage multiple.

22. Dispositif médical selon l'une quelconque des revendications précédentes, **caractérisé par le fait qu'**il comporte en outre un deuxième capteur de pression (8) se situant sur le segment de tubulure patient qui se trouve en aval du premier système d'occlusion (7).

23. Dispositif médical selon les revendications 20 et 22 **caractérisé par le fait qu'** il comporte en outre un appareil qui mesure la différence de pression entre le segment intermédiaire et la tubulure patient par la mesure de la différence des pressions mesurées par les premier et deuxième capteurs de pression (5, 8).

24. Dispositif médical selon l'une quelconque des revendications 20 à 23, **caractérisé par le fait qu'**il comporte en outre une alarme associée au premier capteur de pression (5) et qui se déclenche, en absence d'injection, si le premier capteur de pression (5) mesure une pression de liquide qui diminue dans le segment intermédiaire ou qui descend au-dessous d'une valeur prédéterminée.

25. Dispositif médical selon la revendication 24, **caractérisé par le fait que** les moyens de pompage (2) sont activés pour que l'injection s'enclenche lorsque l'alarme est déclenchée afin de maintenir la pression de liquide du segment intermédiaire supérieure ou égale à un seuil de pression prédéterminé.

26. Dispositif médical selon la revendication 23, **caractérisé par le fait qu'**il comporte en outre une alarme associée audit appareil, ladite alarme se déclenchant lorsque la différence de pression mesurée par ledit appareil devient supérieure ou égale à une valeur prédéterminée.

27. Dispositif médical selon l'une des revendications 20 à 23, **caractérisé par le fait qu'**il comporte en outre une alarme associée au premier capteur de pression (5) et qui se déclenche si le premier capteur de pression (5) mesure une pression de liquide qui augmente dans le segment intermédiaire ou qui monte au-dessus d'une valeur prédéterminée.

28. Dispositif médical selon l'une des revendications 20 à 23, **caractérisé par le fait qu'**il possède des moyens (3) pour modifier la pression du segment intermédiaire en l'absence d'injection afin d'obtenir une pression de liquide dans le segment intermédiaire qui se situe à un seuil déterminé compris entre la pression de tubulure patient et la pression d'ouverture du premier système d'occlusion (7).

29. Dispositif médical selon la revendication 28, **caractérisé par le fait que** les moyens pour modifier la pression sont constitués d'une chambre intermédiaire (3) de volume réglable reliée au segment intermédiaire.

30. Dispositif médical selon la revendication 29, **caractérisé par le fait que** le volume de la chambre intermédiaire (3) peut être réglé par un moyen tel qu'un piston.

31. Dispositif médical selon la revendication 28, **caractérisé par le fait que** les moyens pour modifier la pression du segment intermédiaire sont les moyens de pompage (2).

32. Dispositif médical selon les revendications 14 et 30, **caractérisé en ce que** ledit système de déconnexion (6) comprend une pièce amont (10) et une pièce aval (14) pouvant coopérer de façon amovible pour former une liaison étanche, ladite pièce amont (10) comprenant un volume interne réglable au moyen d'un piston mobile (20).

33. Dispositif médical selon la revendication 32, **caractérisé en ce que** le volume interne de ladite pièce amont (10) est conformé pour permettre l'obtention d'une pression de liquide dans le système de déconnexion (6) qui soit inférieure ou égale à la pression atmosphérique avant la séparation complète entre lesdites pièces amont et aval (10, 14).

34. Dispositif médical selon l'une quelconque des revendications précédentes, **caractérisé par le fait que** lesdits moyens de pompage (2) comprennent un pousse-seringue muni d'une seringue avec un piston mobile.

35. Dispositif médical selon la revendication 34, **caractérisé par le fait que** le volume interne de la seringue fait partie intégrante du segment intermédiaire.

36. Dispositif médical selon la revendication 35, **caractérisé par le fait qu'**il comporte en outre un capteur de pression qui se situe sur l'extrémité distale du piston du pousse-seringue.

37. Dispositif médical selon la revendication 35, **caractérisé en ce que** la seringue forme ledit système de régulation (4).

38. Dispositif médical selon les revendications 28 et 35, **caractérisé en ce que** la seringue forme lesdits moyens (3) pour modifier la pression en liquide du segment intermédiaire.

39. Dispositif médical selon la revendication 14 et l'une des revendications 24 à 27, **caractérisé par le fait que** l'alarme se désactive lorsque la tubulure à usage unique est déconnectée.

40. Dispositif médical selon l'une quelconque des revendications 1 à 39, **caractérisé par le fait qu'**il comporte en outre un deuxième système de déconnexion situé en amont du système de régulation (4), ledit deuxième système de déconnexion permettant ainsi le changement du segment intermédiaire en cas de risque de contamination.

41. Dispositif médical selon l'une quelconque des revendications 1 à 39 et 40, **caractérisé par le fait que** le premier système d'occlusion (7) ou le système de régulation (4), ou les deux, sont de type clamp automatique.

42. Dispositif médical selon l'une quelconque des revendications 24 à 27, **caractérisé par le fait qu'**il comporte en outre un quatrième système d'occlusion qui est activé en cas de déclenchement de l'alarme.

43. Dispositif médical selon les revendications 14 et 42, **caractérisé par le fait que** le quatrième système d'occlusion est placé en aval du système de déconnexion (6).

44. Dispositif médical selon l'une quelconque des revendications précédentes, **caractérisé en ce qu'**il comprend en outre un capteur de pression mesurant la pression de liquide dans un tronçon de la tubulure situé en amont du segment intermédiaire.

## Patentansprüche

1. Medizinische Vorrichtung zum Injizieren von Flüssigkeit unter Druck, umfassend eine Flüssigkeitsquelle (1), Pumpmittel (2), einen Schlauch, auf dem sich mindestens ein erstes Flüssigkeitsokklusionssystem, das einen ersten Öffnungsdruck (P1) aufweist und ein Ventil (7) oder eine Klammer umfaßt, und ein Regelsystem (4), das einen zweiten Öffnungsdruck (P2) aufweist und oberhalb des ersten Okklusionssystems angeordnet ist, befinden, sowie Injektionsmittel, die mit einem Patienten verbindbar sind,
**dadurch gekennzeichnet, daß**
das Regelsystem (4) und das erste Flüssigkeitsokklusionssystem (7) einen Zwischenabschnitt festlegen, in dem der Druck, wenn keine Injektion vorliegt, immer über dem Druck gehalten wird, der in dem ersten Okklusionssystems (7) herrscht, wobei der erste Öffnungsdruck (P1) kleiner ist als der zweite Öffnungsdruck (P2), und der Druck des Zwischenabschnitts kleiner ist als der erste Öffnungsdruck (P1), wenn keine Injektion vorliegt, um einen Flüssigkeitsrückfluß in Richtung auf den Zwischenabschnitt an dem ersten Okklusionssystem (7) zu verhindern.

2. Medizinische Vorrichtung zum Injizieren von Flüssigkeit nach Anspruch 1, **dadurch gekennzeichnet, daß** ein Auslaufen von Flüssigkeit aus dem Zwischenabschnitt durch Messen der damit verbundenen Druckverminderung erfaßt wird.

3. Medizinische Vorrichtung nach einem der Ansprüche 1 und 2, **dadurch gekennzeichnet, daß** das Ventil oder die Klammer des ersten Flüssigkeitsokklusionssystems ein Rückschlagventil (7) ist.

4. Medizinische Vorrichtung nach Anspruch 3, **dadurch gekennzeichnet, daß** das Rückschlagventil (7) einen Stopfen (38) umfaßt, der sich radial zusammendrücken läßt, wobei er seine Umgehung durch die Flüssigkeit ermöglicht, wenn der Flüssigkeitsdruck oberhalb des Stopfens (38) größer als oder gleich dem Öffnungsdruck des Rückschlagventils (7) ist.

5. Medizinische Vorrichtung nach einem der Ansprüche 3 oder 4, **dadurch gekennzeichnet, daß** das Rückschlagventil (7) einen Öffnungsdruck aufweist, der größer ist als sein Schließdruck.

6. Medizinische Vorrichtung nach einem der Ansprüche 1 bis 5, **dadurch gekennzeichnet, daß** das Regelsystem (4) ein zweites Okklusionssystem umfaßt.

7. Medizinische Vorrichtung nach Anspruch 6, **dadurch gekennzeichnet, daß** die ersten und zweiten Okklusionssysteme (4, 7) unterschiedliche Öffnungsdrücke aufweisen.

8. Medizinische Vorrichtung nach Anspruch 7, **dadurch gekennzeichnet, daß** der Öffnungsdruck des zweiten Okklusionssystems (4) größer ist als der Öffnungsdruck des ersten Okklusionssystems (7), um ständig einen Flüssigkeitsfluß nach unten sicherzustellen.

9. Medizinische Vorrichtung nach einem der Ansprüche 6 bis 8, **dadurch gekennzeichnet, daß** das zweite Okklusionssystem (4) ein System zum Zusammendrücken des Schlauchs ist.

10. Medizinische Vorrichtung nach Anspruch 9, **dadurch gekennzeichnet, daß** das System zum Zusammendrücken des Schlauchs aus den Rollen einer peristaltischen Kassette besteht.

11. Medizinische Vorrichtung nach einem der Ansprüche 6 bis 8, **dadurch gekennzeichnet, daß** das zweite Okklusionssystem (4) ein Rückschlagventil umfaßt.

12. Medizinische Vorrichtung nach Anspruch 11, **dadurch gekennzeichnet, daß** das Rückschlagventil (7) einen Stopfen (38) umfaßt, der sich radial zusammendrücken läßt, wobei er seine Umgehung durch die Flüssigkeit ermöglicht, wenn der Flüssigkeitsdruck oberhalb des Stopfens (38) größer als oder gleich einem vorherbestimmten Öffnungsdruck ist.

13. Medizinische Vorrichtung nach einem der vorhergehenden Ansprüche, **dadurch gekennzeichnet, daß** der Öffnungsdruck des ersten Okklusionssystems (7) größer ist als der Druck, der in dem Zwischenabschnitt herrscht, wenn keine Injektion vorliegt.

14. Medizinische Vorrichtung nach einem der vorhergehenden Ansprüche, **dadurch gekennzeichnet, daß** sie außerdem ein Trennsystem (6) umfaßt, das auf dem Zwischenabschnitt liegt, wobei das Trennsystem (6) unterhalb einen Einwegund oberhalb einen Mehrwegschlauch festlegt.

15. Medizinische Vorrichtung nach Anspruch 14, **dadurch gekennzeichnet, daß** das Trennsystem (6) mit Okklusionsmitteln (7, 20) des Schlauchs versehen ist, die sich vor dem Trennen einschalten.

16. Medizinische Vorrichtung nach Anspruch 15, **dadurch gekennzeichnet, daß** das Trennsystem zwei einander gegenüber codierte Teile umfaßt, welche das Ein- oder Ausschalten der Okklusionsmittel nur dann erlauben, wenn die Codierung eines Teils gegenüber dem anderen gewahrt ist.

17. Medizinische Vorrichtung nach Anspruch 16, **dadurch gekennzeichnet, daß** die Codierung von der Art Schlüssel-Schloß ist.

18. Medizinische Vorrichtung nach Anspruch 17, **dadurch gekennzeichnet, daß** das Ein- bzw. Ausschalten der Okklusionsmittel durch eine Drehung sichergestellt wird, so daß das Trennen nur erfolgen kann, wenn der Schlüssel derart in dem Schloß gedreht wird, daß die Okklusionsmittel abgeschaltet werden, und daß die Rückkehr des Schlüssels in die Freigabestellung das Verschließen der Okklusionsmittel zur Folge hat.

19. Medizinische Vorrichtung nach Anspruch 15, **dadurch gekennzeichnet, daß** das Trennsystem (6) zwei Teile (10, 14) umfaßt, die jeweils mit Okklusionsmitteln (20, 7) ausgestattet sind, wobei die Okklusionsmittel eines der beiden Teile mindestens von der Art einer Klappe (7) sind und sich automatisch öffnen, wenn die beiden Teile (10, 14) hermetisch in Eingriff gebracht werden, und daß der obere Flüssigkeitsdruck größer ist als der Öffnungsdruck der Klappe (7).

20. Medizinische Vorrichtung nach einem der vorhergehenden Ansprüche, **dadurch gekennzeichnet, daß** ein erster Druckgeber (5) den Flüssigkeitsdruck und/oder die Veränderung des Flüssigkeitsdrucks an dem Zwischenabschnitt messen kann.

21. Medizinische Vorrichtung nach Anspruch 20 und einem der Ansprüche 14 oder 15, **dadurch gekennzeichnet, daß** der Druckgeber (5) oberhalb des Trennsystems (6) liegt, um mit einem Teil des Mehrwegschlauchs in Kontakt zu stehen.

22. Medizinische Vorrichtung nach einem der vorhergehenden Ansprüche, **dadurch gekennzeichnet, daß** sie außerdem einen zweiten Druckgeber (8) umfaßt, der auf dem Patientenschlauchabschnitt liegt, der sich oberhalb des ersten Okklusionssystems (7) befindet.

23. Medizinische Vorrichtung nach Anspruch 20 und 22, **dadurch gekennzeichnet, daß** sie außerdem ein Gerät umfaßt, das den Druckunterschied zwischen dem Zwischenabschnitt und dem Patientenschlauch durch Messen des Unterschieds der gemessenen Drücke durch die ersten und zweiten Druckgeber (5, 8) mißt.

24. Medizinische Vorrichtung nach einem der vorhergehenden Ansprüche 20 bis 23, **dadurch gekennzeichnet, daß** sie außerdem einen Alarm umfaßt, der zu dem ersten Druckgeber (5) gehört und ausgelöst wird, wenn keine Injektion vorliegt, falls der erste Druckgeber (5) einen Flüssigkeitsdruck mißt, der in dem Zwischenabschnitt abnimmt oder der unter einen vorherbestimmten Wert abfällt.

25. Medizinische Vorrichtung nach Anspruch 24, **dadurch gekennzeichnet, daß** die Pumpmittel (2) eingeschaltet werden, damit die Injektion einsetzt, wenn der Alarm ausgelöst wird, um den Flüssigkeitsdruck des Zwischenabschnitts größer als oder gleich einer vorherbestimmten Druckschwelle zu halten.

26. Medizinische Vorrichtung nach Anspruch 23, **dadurch gekennzeichnet, daß** sie außerdem einen Alarm umfaßt, der zu dem Gerät gehört, wobei der Alarm ausgelöst wird, wenn der von dem Gerät gemessene Druckunterschied größer als oder gleich einem vorherbestimmten Wert wird.

27. Medizinische Vorrichtung nach einem der Ansprüche 20 bis 23, **dadurch gekennzeichnet, daß** sie außerdem einen Alarm umfaßt, der zu dem ersten Druckgeber (5) gehört und ausgelöst wird, wenn der erste Druckgeber (5) einen Flüssigkeitsdruck mißt, der in dem Zwischenabschnitt zunimmt oder über einen vorherbestimmten Wert hinaus ansteigt.

28. Medizinische Vorrichtung nach einem der Ansprüche 20 bis 23, **dadurch gekennzeichnet, daß** sie Mittel (3) besitzt, um den Druck des Zwischenabschnitts zu ändern, wenn keine Injektion vorliegt, um einen Flüssigkeitsdruck in dem Zwischenabschnitt zu erreichen, der auf einer bestimmten Schwelle liegt, die sich zwischen dem Patientenschlauchdruck und dem Öffnungsdruck des ersten Okklusionssystems (7) befindet.

29. Medizinische Vorrichtung nach Anspruch 28, **dadurch gekennzeichnet, daß** die Mittel zum Ändern des Drucks aus einer Zwischenkammer (3) mit einstellbarem Volumen bestehen, die mit dem Zwischenabschnitt verbunden ist.

30. Medizinische Vorrichtung nach Anspruch 29, **dadurch gekennzeichnet, daß** das Volumen der Zwischenkammer (3) durch Mittel wie etwa ein Kolben einstellbar ist.

31. Medizinische Vorrichtung nach Anspruch 28, **dadurch gekennzeichnet, daß** die Mittel zum Ändern des Drucks des Zwischenabschnitts Pumpmittel (2) sind.

32. Medizinische Vorrichtung nach Anspruch 14 und 30, **dadurch gekennzeichnet, daß** das Trennsystem (6) ein oberes Stück (10) und ein unteres Stück (14) umfaßt, die abnehmbar zusammenwirken können, um eine hermetische Verbindung zu bilden, wobei das obere Stück (10) ein Innenvolumen umfaßt, das mittels eines beweglichen Kolbens (20) einstellbar ist.

33. Medizinische Vorrichtung nach Anspruch 32, **dadurch gekennzeichnet, daß** das Innenvolumen des oberen Stücks (10) angepaßt ist, um einen Flüssigkeitsdruck in dem Trennsystem (6) erhalten zu können, der kleiner als oder gleich dem Luftdruck vor der vollständigen Trennung zwischen den oberen und unteren Stücken (10, 14) ist.

34. Medizinische Vorrichtung nach einem der vorhergehenden Ansprüche, **dadurch gekennzeichnet, daß** die Pumpmittel (2) einen Spritzenschieber umfassen, der mit einer Spritze mit einem beweglichen Kolben versehen ist.

35. Medizinische Vorrichtung nach Anspruch 34, **dadurch gekennzeichnet, daß** das Innenvolumen der Spritze mit dem Zwischenabschnitt einstückig ist.

36. Medizinische Vorrichtung nach Anspruch 35, **dadurch gekennzeichnet, daß** sie außerdem einen Druckgeber umfaßt, der auf dem distalen Ende des Spritzenschieberkolbens liegt.

37. Medizinische Vorrichtung nach Anspruch 35, **dadurch gekennzeichnet, daß** die Spritze das Regelsystem (4) bildet.

38. Medizinische Vorrichtung nach Anspruch 28 und 35, **dadurch gekennzeichnet, daß** die Spritze die Mittel (3) bildet, um den Flüssigkeitsdruck des Zwischenabschnitts zu ändern.

39. Medizinische Vorrichtung nach Anspruch 14 und einem der Ansprüche 24 bis 27, **dadurch gekennzeichnet, daß** der Alarm ausgeschaltet wird, wenn der Einwegschlauch abgetrennt wird.

40. Medizinische Vorrichtung nach einem der Ansprüche 1 bis 39, **dadurch gekennzeichnet, daß** sie außerdem ein zweites Trennsystem umfaßt, das oberhalb des Regelsystems (4) liegt, wobei das zweite Trennsystem somit die Änderung des Zwischenabschnitts im Falle eines Kontaminationsrisikos ermöglicht.

41. Medizinische Vorrichtung nach einem der Ansprüche 1 bis 39 und 40, **dadurch gekennzeichnet, daß** das erste Okklusionssystem (7) oder das Regelsystem (4), oder beide, von der Art einer automatischen Klammer sind.

42. Medizinische Vorrichtung nach einem der Ansprüche 24 bis 27, **dadurch gekennzeichnet, daß** sie außerdem ein viertes Okklusionssystem umfaßt, das eingeschaltet wird, falls der Alarm ausgelöst wird.

43. Medizinische Vorrichtung nach Anspruch 14 und 42, **dadurch gekennzeichnet, daß** das vierte Okklusionssystem unterhalb des Trennsystems (6) angeordnet wird.

44. Medizinische Vorrichtung nach einem der vorhergehenden Ansprüche, **dadurch gekennzeichnet, daß** sie außerdem einen Druckgeber umfaßt, der den Flüssigkeitsdruck in einem Teilstück des Schlauchs mißt, das oberhalb des Zwischenabschnitts liegt.

## Claims

1. A medical device for injecting liquid under pressure, comprising a liquid source (1), pump means (2), a piece of tubing on which there is situated at least a first liquid occlusion system having a first opening pressure (P1) and comprising a valve or clamp (7) and a regulation system (4) having a second opening pressure (P2) and placed upstream from the first occlusion system, and injection means which can be connected to a patient, the medical device being **characterized by** the fact that the regulation system (4) and the first liquid occlusion system (7) define an intermediate segment in which the pressure, in the absence of injection, is always maintained greater than the pressure that exists downstream from the first occlusion system (7), said first opening pressure (P1) being lower than said second opening pressure (P2) and the pressure in the intermediate segment being lower than said first opening pressure (P1) in the absence of injection, so as to prevent any liquid from flowing back towards the intermediate segment in the first occlusion system (7).

2. A medical device for injecting liquid according to claim 1, **characterized by** the fact that any leakage of liquid from the intermediate segment can be detected by measuring the associated drop in pressure.

3. A medical device according to claim 1 or 2, **characterized by** the fact that said valve or clamp of the first liquid occlusion system is a non-return valve (7).

4. A medical device according to claim 3, **characterized in that** the non-return valve (7) comprises a plug (38) capable of being compressed radially so as to allow liquid to bypass it in the event of the pressure of the liquid upstream from the plug (38) being greater than or equal to the opening pressure of the non-return valve (7).

5. A medical device according to claim 3 or 4, **characterized in that** the non-return valve (7) has an opening pressure that is greater than its closing pressure.

6. A medical device according to any one of claims 1 to 5, **characterized by** the fact that the regulation system (4) includes a second occlusion system.

7. A medical device according to claim 6, **characterized by** the fact that the first and second occlusion systems (4, 7) have different opening pressures.

8. A medical device according to claim 7, **characterized by** the fact that the opening pressure of the second occlusion system (4) is greater than the opening pressure of the first occlusion system (7) so as to ensure that liquid flows permanently downstream.

9. A medical device according to any one of claims 6 to 8, **characterized by** the fact that the second occlusion system (4) is a system involving flattening of the tubing.

10. A medical device according to claim 9, **characterized by** the fact that the system for flattening the tubing is constituted by the wheels of a peristaltic cassette.

11. A medical device according to any one of claims 6 to 8, **characterized by** the fact that the second occlusion system (4) has a non-return valve.

12. A medical device according to claim 11, **characterized in that** the non-return valve (7) comprises a plug (38) capable of being compressed radially so as to enable it to be bypassed by the liquid when the pressure of the liquid upstream from the plug (38) is greater than or equal to a predetermined opening pressure.

13. A medical device according to any one of the preceding claims, **characterized by** the fact that the opening pressure of the first occlusion system (7) is greater than the pressure that exists in the intermediate segment in the absence of injection.

14. A medical device according to any one of the preceding claims, **characterized by** the fact that it further comprises a disconnection system (6) situated on the intermediate segment, the disconnection system (6) defining a single-use tubing downstream and a multiple-use tubing upstream.

15. A medical device according to claim 14, **characterized by** the fact that the disconnection system (6) is provided with means (7, 20) for occluding the tubing which are activated prior to disconnection.

16. A medical device according to claim 15, **characterized by** the fact that the disconnection system comprises two mutually encoded pieces which enable the occlusion means to be activated or deactivated only if the coding of one piece complies with that of the other.

17. A medical device according to claim 16, **characterized by** the fact that the encoding is of the key-and-lock type.

18. A medical device according to claim 17, **characterized by** the fact that activation and deactivation of the occlusion means is provided by rotation such that disconnection cannot take place when the key is turned in the lock in such a manner that the occlusion means are deactivated and that return of the key into the release position causes the occlusion means to be closed.

19. A medical device according to claim 15, **characterized by** the fact that the disconnection system (6) includes two pieces (10, 14) each of which is equipped with occlusion means (20, 7), the occlusion means of at least one of the two pieces being of the check valve type (7) that opens automatically when the two pieces (10, 14) are engaged in sealed manner and when the pressure in the liquid upstream is greater than the opening pressure in said check valve (7).

20. A medical device according to any one of the preceding claims, **characterized by** the fact that a first pressure sensor (5) makes it possible to measure the liquid pressure and/or the liquid pressure variation in the intermediate segment.

21. A medical device according to claim 20 and one of claims 14 or 15, **characterized by** the fact that the pressure sensor (5) is located upstream from the disconnection system (6) so as to be in contact with a portion of the multiple-use tubing.

22. A medical device according to any one of the preceding claims, **characterized by** the fact that it further includes a second pressure sensor (8) that is situated on the patient tubing segment located downstream from the first occlusion system (7).

23. A medical device according to claims 20 and 22, **characterized by** the fact that it further includes an apparatus for measuring the difference in pressure between the intermediate segment and the patient tubing by measuring the difference in the pressures as measured by the first and second pressure sensors (5, 8).

24. A medical device according to any one of claims 20 to 23, **characterized by** the fact that it further includes an alarm that is associated with the first pressure sensor (5) and that is triggered, in the absence of injection, if the first pressure sensor (5) measures a liquid pressure that decreases in the intermediate segment or that drops beneath a predetermined value.

25. A medical device according to claim 24, **characterized by** the fact that the pump means (2) are activated so that injection is engaged when the alarm is triggered so as to maintain the liquid pressure in the intermediate segment greater than or equal to a predetermined threshold.

26. A medical device according to claim 23, **characterized by** the fact that it further includes an alarm that is associated with said apparatus, said alarm being triggered when the pressure difference as measured by said apparatus becomes greater than or equal to a predetermined value.

27. A medical device according to one of claims 20 to 23, **characterized by** the fact that it further includes an alarm that is associated with the first pressure sensor (5) and that is triggered if the first pressure sensor (5) measures a liquid pressure that increases in the intermediate segment or that rises above a predetermined value.

28. A medical device according to one of claims 20 to 23, **characterized by** the fact that it possesses means (3) for modifying the pressure of the intermediate segment in the absence of injection so as to obtain a liquid pressure in the intermediate segment which lies at a determined threshold between the patient tubing pressure and the opening pressure of the first occlusion system (7).

29. A medical device according to claim 28, **characterized by** the fact that the means for modifying pressure are constituted by an intermediate chamber (3) of adjustable volume that is connected to the intermediate segment.

30. A medical device according to claim 29, **characterized by** the fact that the volume of the intermediate chamber (3) can be adjusted by means such as a piston.

31. A medical device according to claim 28, **characterized by** the fact that the means for modifying pressure in the intermediate segment are the pump means (2).

32. A medical device according to claims 14 and 30, **characterized in that** said disconnection system (6) comprises an upstream piece (10) and a downstream piece (14) capable of co-operating in releasable manner so as to form a leakproof link, said upstream piece (10) having an internal volume that is adjustable by means of a moving piston (20).

33. A medical device according to claim 32, **characterized in that** the internal volume of said upstream piece (10) is shaped to enable liquid pressure to be obtained in the disconnection system (6) which is lower than or equal to atmospheric pressure, prior to said upstream and downstream pieces (10, 14) being fully separated.

34. A medical device according to any one of the preceding claims, **characterized by** the fact that said pump means (2) comprise a syringe pusher provided with a syringe having a moving piston.

35. A medical device according to claim 34, **characterized by** the fact that the internal volume of the syringe forms an integral portion of the intermediate segment.

36. A medical device according to claim 35, **characterized by** the fact that it further includes a pressure sensor that is situated at the distal end of the syringe pusher piston.

37. A medical device according to claim 35, **characterized in that** the syringe forms said regulation system (4).

38. A medical device according to claims 28 and 35, **characterized in that** the syringe forms said means (3) for modifying the liquid pressure of the intermediate segment.

39. A medical device according to claim 14 and one of claims 24 to 27, **characterized by** the fact that the alarm deactivates when the single-use tubing is disconnected.

40. A medical device according to any one of claims 1 to 39, **characterized by** the fact that it further includes a second disconnection system that is situated upstream from the regulation system (4), with said second disconnection system thus enabling the intermediate segment to be changed in the event of a risk of contamination.

41. A medical device according to any one of claims 1 to 39 and 40, **characterized by** the fact that the first occlusion system (7), or the regulation system (4), or both of them, are of the automatic clamp type.

42. A medical device according to any one of claims 24 to 27, **characterized by** the fact that it further includes a fourth occlusion system that is activated in the event of the alarm being triggered.

43. A medical device according to claims 14 and 42, **characterized by** the fact that the fourth occlusion system is placed downstream from the disconnection system (6).

44. A medical device according to any one of the preceding claims, **characterized in that** it further comprises a pressure sensor measuring the pressure of the liquid in a length of the tubing situated upstream from the intermediate segment.
